(19) Europäisches Patentamt
European Patent Office
Office européen des brevets

(11) **EP 4 296 255 A1**

(12) **EUROPEAN PATENT APPLICATION**
published in accordance with Art. 153(4) EPC


(43) Date of publication:
**27.12.2023 Bulletin 2023/52**

(21) Application number: **21926342.3**

(22) Date of filing: **15.11.2021**

(51) International Patent Classification (IPC):
***C07C 51/27*** *(2006.01)* ***C07C 249/10*** *(2006.01)*
***C07C 55/14*** *(2006.01)* ***C07C 251/44*** *(2006.01)*

(52) Cooperative Patent Classification (CPC):
Y02P 20/30

(86) International application number:
**PCT/CN2021/130661**

(87) International publication number:
**WO 2022/174628 (25.08.2022 Gazette 2022/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.02.2021 CN 202110197792**



(71) Applicant: **Xiangtan University**
**Xiangtan, Hunan 411105 (CN)**

(72) Inventors:
- **LUO, He'an**
  **Xiangtan, Hunan 411105 (CN)**
- **YOU, Kuiyi**
  **Xiangtan, Hunan 411105 (CN)**
- **JIAN, Jian**
  **Xiangtan, Hunan 411105 (CN)**
- **LIU, Pingle**
  **Xiangtan, Hunan 411105 (CN)**
- **NI, Wenjin**
  **Xiangtan, Hunan 411105 (CN)**
- **AI, Qiuhong**
  **Xiangtan, Hunan 411105 (CN)**

(74) Representative: **Vitina, Maruta et al**
**Agency TRIA ROBIT**
**P.O. Box 22**
**1010 Riga (LV)**


# (54) METHOD FOR CO-PRODUCING ADIPIC ACID AND CYCLOHEXANONE OXIME FROM CYCLOHEXANE


(57) A method for producing adipic acid and cyclohexanone oxime from cyclohexane is provided. Cyclohexane and $NO_x$ undergo oxidation-nitration reaction to produce a mixture of adipic acid, nitrocyclohexane, nitrogen oxides and by-product A, from which adipic acid and nitrocyclohexane are separated. The nitrocyclohexane is catalytically hydrogenated with hydrogen to produce cyclohexanone oxime and a small amount of cyclohexylamine, where cyclohexanone oxime is collected, and the cyclohexylamine is partially oxidized with molecular oxygen to obtain cyclohexanone oxime and by-product B. Without separation, or after removing part or all of water from the reaction mixture, under the action of a catalyst, the reaction mixture undergoes hydrogenation and amination simultaneously or sequentially, or the hydrogenation alone, and separation to give cyclohexanone oxime.


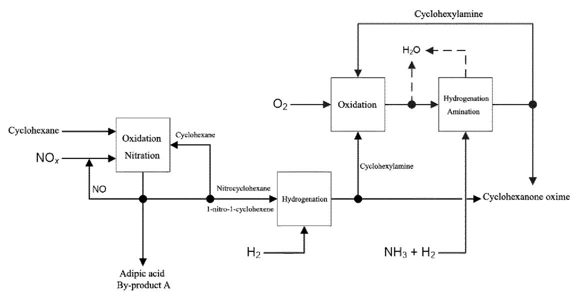


Fig. 1

EP 4 296 255 A1

## Description

## TECHNICAL FIELD

This application relates to production of adipic acid and cyclohexanone oxime, and more particularly to a method for producing adipic acid and cyclohexanone oxime from cyclohexane.

## BACKGROUND

Adipic acid is a very important industrial material and intermediate, and mainly used in the manufacturing of nylon 66, greases, pesticides, adhesives and so on.

Cyclohexanone is an intermediate of an important raw material ε-caprolactam (mainly used to generate polyamide slices by polymerization to further manufacture polyamide fiber, engineering plastics, plastic films and so on).

At present, the industrial production of adipic acid generally adopts a two-step oxidation method with cyclohexane as raw material: firstly, cyclohexane is oxidized by air to prepare KA oil (a mixture of cyclohexanone and cyclohexanol), and then the KA oil is oxidized by nitric acid to obtain adipic acid. In cyclohexane oxidization, both single-pass conversion and KA oil yield are very low, which are generally 3~5% and 82~83% respectively; besides, single-pass conversion and adipic acid yield are also not high, which are generally 5~12% and 90~94% respectively. The amount of nitric acid id large, each 1 ton of products needs to consume about 1.3 tons of 68% nitric acid, and produce a lot of waste acid, wastewater and waste gas, such as CO and $N_2O$. Therefore, though the two-step oxidation of cyclohexane is relatively mature, and has been widely used in industrial production, it still struggles with high energy consumption, serious environmental pollution, low resource utilization efficiency and high production cost.

A two-step process of hydration and oxidation of cyclohexene is a new method in recent years. Firstly, cyclohexene is hydrated as cyclohexanol, and part of cyclohexanol is dehydrogenated to cyclohexanone to form KA oil. Then, cyclohexanol is oxidized by nitric acid to obtain adipic acid. The advantage of this method is high selectivity of first step, but second step still has some problems such as high energy consumption and great environmental impact. In addition, in this process, the cyclohexene is obtained from selectively partial hydrogenation of benzene, which the conditions of reaction and separation are demanding and energy consumption is high.

The one-step synthesis of adipic acid through oxidation of cyclohexene with hydrogen peroxide is an environmentally friendly process, in which the cyclohexene is oxidized by hydrogen peroxide into adipic acid in the presence of a catalyst. However, the catalyst involved in such synthesis process, such as methyl trioctyl ammonium chloride, sodium tungstate-oxalic acid system and sodium tungstate-sulfuric acid system, is relatively expensive, and the cyclohexene preparation requires harsh reaction and separation conditions, resulting in high production costs. Moreover, this synthesis process also involves large consumption of hydrogen peroxide. Therefore, its industrial application is limited.

Bio-oxidation process is based on an enzyme encoded by a gene cluster separated from an aerobic denitrifying bacterial strain, which can convert cyclohexanol to adipic acid in a suitable growth condition. However, this process is expensive and not suitable for large-scale industrial production.

One step synthesis of adipic acid by direct oxidation of cyclohexane with molecular oxygen is always expected. As early as 1940s, US Patent Application Publication No. 2223493A (1940) presented a direct synthesis of adipic acid by air oxidation of cyclohexane with acetic acid as solvent and soluble transition metal salts (cobalt, copper, manganese, etc.) as catalyst. Based on the above method, US Patent Application Publication No. 4263453A (1981) suggested that increasing the amount of acetic acid solvent (a mole ratio of cyclohexane to acetic acid is 1:6) and introducing a small amount of butanone and water can improve the conversion efficiency of cyclohexane and the selectivity of adipic acid; US Patent Application Publication No. 5321157A (1994) proposed that using oxygen-enriched air can reduce the amount of acetic acid solvent (the mole ratio of cyclohexane to acetic acid is close to 1:1) and obtain relatively high selectivity of adipic acid. Though, these methods can obtain a relatively high conversion efficiency of cyclohexane and selectivity of adipic acid, there are also many problems. Firstly, under this reaction temperature and pressure conditions, acetic acid is more corrosive to equipment; secondly, target product, adipic acid, and other by-products are hard to be separated and purified from acetic acid solvent; thirdly, soluble catalyst is hard to be separated from the acetic acid solvent, though French Patent No. 2722783B (1996) and French Patent No. 2746671B (1997) presented a method for recycling catalyst, their process is complex and their cost is high. Therefore, the synthesis of adipic acid by direct oxidation of cyclohexane with molecular oxygenin an acetic acid solvent is hard to be industrialized.

US Patent Application Publication No.0147777A1(2004) presented a synthesis of KA oil and adipic acid by air oxidation of cyclohexane with caprylic acid as solvent, cobalt acetylacetonate as catalyst and compounds containing imide function as promotor; US Patent No. 7507856B2 (2009) presented a synthesis of adipic acid by air oxidation of cyclohexane with 4-tert-butylbenzoic acid as solvent, manganese acetylacetonate as catalyst and cyclohexanone as inducer, which the conversion efficiency of cyclohexane and the selectivity of adipic acid can reach 7.17% and 53.6%

respectively; US Patent Application Publication No. 0095258A1 (2012) presented a synthesis of adipic acid by oxidation of cyclohexane with molecular oxygen, acetonitrile as solvent and metal oxides ($TiO_2$, $ZrO_2$, MgO, etc.) loaded with precious metals (Au, Pd, Pt, etc.) as catalyst, which the conversion efficiency of cyclohexane and the selectivity of adipic acid are 25% and 26% respectively. These methods can avoid relatively more serious problems of corrosivity of acetic acid, but the conversion efficiency of cyclohexane and the selectivity of adipic acid are low and there are still problems such as difficult separation of solvents and products.

For more than a decade, the research on preparation of adipic acid with molecular oxygen as oxidant has focused on solvent-free condition. Raja, Sankar and Thomas (J. Am. Chem. Soc.,1999) reported a catalyst, aluminophosphate molecular sieves loaded with transition metal (Fe, Mn, Co, etc.) catalyzes oxidation of cyclohexane in solvent-free condition, among which FeAlPO-5 had the best catalytic effect, the conversion efficiency of cyclohexane was 19.8%, the selectivity of adipic acid was 32.3%, and the other main substances were cyclohexanol, cyclohexanone and some lipids. Yuan et al. (Organic Process R&D, 2004) reported research on a synthesis of adipic acid by catalytic oxidation of cyclohexane with metalloporphyrin as catalyst in solvent-free condition, and adipic acid yield was 21.4%. Recently, Lv, Ren and Liu (Applied Catalysis A, 2012) reported research on a synthesis of adipic acid by catalytic oxidation of cyclohexane with Anderson-type catalyst $[(C_{18}H_{37})_2N(CH_3)_2]_6Mo_7O_{24}$ in solvent-free condition, which the conversion efficiency of cyclohexane was 10.2% and the selectivity of adipic acid was 87.1%.

At present, there are two main methods for industrial production of cyclohexanone oxime: cyclohexanone-hydroxylamine method and ammoximation of cyclohexanone method, both of which synthesize cyclohexanone oxime through an intermediate cyclohexanone from benzene.

There are three methods for industrial production of cyclohexanone from benzene: phenol method, oxidation of cyclohexane method and hydration of cyclohexene method. The earliest production unit of cyclohexanone oxime in the world all adopted the phenol process to produce cyclohexanone: firstly, phenol is produced from benzene; then, cyclohexanol is produced by hydrogenation of phenol; and then, cyclohexanone is produced by dehydrogenation of cyclohexanol. The key of phenol process is how to produce phenol. At present, industries produce phenol mainly by isopropylbenzene (hydrolysis of chlorobenzene and sulfonation of benzene are almost all eliminated owing to environmental and cost problems): firstly, isopropylbenzene is produced by alkylation of benzene and propylene; then isopropylbenzene is reacted with oxygen to produce cumene hydroperoxide; finally, cumene hydroperoxide is decomposed into phenol and acetone under the action of sulfuric acid and sulfonic acid resin. The main disadvantages of this method are as follows: the phenol yield is low (72~75%) and the by-products are much; the separation and purification equipment of phenol and acetone is complex and its energy consumption is high; and the market need and price of a large number of acetone of by-products will affect the cost of phenol. Therefore, the synthesis of cyclohexanone from phenol was gradually replaced by oxidation of cyclohexane at a early stage.

The technology of a synthesis of cyclohexanone by oxidation of cyclohexane with molecular oxygen is relatively mature. At present, a two-step synthesis method is widely adopted in industries: (i) cyclohexane is oxidized with molecular oxygen in catalyst-free condition to generate cyclohexyl hydroperoxide, a certain amount of cyclohexanone and cyclohexanol and some by-products; (ii) the cyclohexyl hydroperoxide in the oxidation product obtained in the previous step is decomposed into cyclohexanone and cyclohexanol (together with some by-products) by low-temperature alkaline decomposition, and then KA oil is obtained by separation; then KA oil is further separated into cyclohexanone and cyclohexanol, and finally cyclohexanone is produced by dehydrogenation of cyclohexanol. The main advantages of this method are as follows: the technology of synthesis of cyclohexane by complete hydrogenation of benzene is mature, and has low difficulty and high yield. But the oxidation process of cyclohexane has three main disadvantages: (i) In order to maintain relatively high selectivity, the single-pass conversion of cyclohexane of air oxidation of cyclohexane can only be controlled at 3-5%, and a large amount of unconverted cyclohexane requires relatively high energy to be separated and cycled. However, the total yield of KA oil (a mixture of cyclohexanone and cyclohexanol) is only about 83% measured by cyclohexane, showing large consumption of cyclohexane and generation of a large number of by-products. (ii) The main product of oxidation of cyclohexane in catalyst-free condition is cyclohexyl hydroperoxide, which needs to consume NaOH to decompose. The by-products of oxidation of cyclohexane are mainly acid, ester, ether and so on, which also need to be saponified and removed by alkaline aqueous solution. In this way, a large amount of NaOH is consumed and a large amount of saponification waste lye is generated, causing high production cost and high environmental pressure. (iii) Because target product is cyclohexanone, KA oil is need to be further separated into cyclohexanone and cyclohexanol by distillation, and then cyclohexanone is produced by dehydrogenation of cyclohexanol; however, the single-pass conversion of dehydrogenation of cyclohexanol is generally less than 80% owing to limitation of thermodynamic equilibrium, cyclohexanone and cyclohexanol need to be separated after dehydrogenation and a boiling point difference between cyclohexanone and cyclohexanol is only about 6°C; as mentioned above that the single-pass conversion of cyclohexane of air oxidation of cyclohexane is only 3-5%, therefore, the entire consumption of the process is also very high.

In conclusion, there are "three high one large" problems, though the two-step process of synthesis of cyclohexanone by oxidation of cyclohexane widely used in industries at present is relatively mature and has low technological

barrier: high cyclohexane consumption, high alkali consumption, high energy consumption and large load of waste lye treatment.

In recent years, the production of cyclohexanone in new cyclohexanone-oxime industrial units generally adopt cyclohexene hydration process proposed by AsahiKASEI in 2002 (Chinese Patent No. 02804368.5 and Chinese Patent No. 02814607.7) : Cyclohexene and cyclohexane are produced by partial hydrogenation of benzene with hydrogen, then cyclohexene is separated from cyclohexane and unconverted benzene by extraction and distillation, then cyclohexanol is produced by hydration of the cyclohexene and water, and finally cyclohexanone is produced by dehydrogenation of cyclohexanol. The most obvious advantage of this method is low material consumption: first, a total selectivity of partial hydrogenation of cyclohexene and cyclohexane is very high (up to 99% and above), and cyclohexane is a product or intermediate with certain economic value; in addition, the synthesis of cyclohexanol by hydration of cyclohexene is basically a directed conversion reaction. However, it also has obvious disadvantage of high energy consumption: (i) To obtain the single-pass conversion of cyclohexene as high as possible, the conversion efficiency of partial hydrogenation of benzene is generally controlled at 40~50% (when the selectivity of cyclohexene is about 70-80%), in this way, the reaction production of the partial hydrogenation of benzene is actually a mixture of benzene, cyclohexene and cyclohexane with very close boiling points. At present, they can separated only by two-stage extractive distillation and decompression distillation : first-stage extractive distillation is that benzene is separated from cyclohexene and cyclohexane by extraction agent, then benzene and extraction agent are separated by decompression distillation and are recycled respectively; second-stage decompression distillation is that cyclohexene is separated from cyclohexane by extraction agent, then cyclohexene and extraction agent are separated by decompression distillation, the separated cyclohexene can be sold as a product after refinement. It shows that the separation of the benzene-cyclohexene-cyclohexane system has high difficulty and high energy consumption. (ii) The conversion efficiency of cyclohexanol by hydration of cyclohexene is generally only 10-12%, therefore, the separation of cyclohexene-cyclohexanol-water system and the recycle of a large amount of cyclohexene are also require a lot of energy. (iii) Owing to target product is cyclohexanone, cyclohexanone is produced by dehydrogenation of the hydrated cyclohexanol; the dehydrogenation process requires energy and the conversion efficiency of cyclohexanol is generally less than 80% because of limitation of chemical equilibrium; the boiling point difference between cyclohexanone and cyclohexanol is only 6°C, so a large amount of energy is required to separate cyclohexanone and cyclohexanol (the obtained cyclohexanol is in recycle dehydrogenation). In addition to the problem of high energy consumption, the cyclohexene hydration process has problems of the market and utilization of cyclohexane as a by-product: at current industrial level, the by-product cyclohexane yield is relatively large, and the cyclohexane yield is generally more than 0.2 tons per ton of cyclohexanone. But the market demand of cyclohexane is limiting, therefore, at present, some enterprises have to set a production line of oxidation of cyclohexane.

In addition to above problems in the production of cyclohexanone oxime with cyclohexanone as intermediate, oximation of cyclohexanone also has relatively big problems. At present, there are two main methods: hydroxylamine oximation of cyclohexanone method and ammoximation of cyclohexanone method. The hydroxylamine oximation of cyclohexanone method includes hydroxylamine sulfate oximation (HSO method) and hydroxylamine phosphate oximation (HPO method). The HSO method and the HPO method require complex production line of hydroxylamine, then the hydroxylamine is utilized for oximation hydroxylamine of cyclohexanone to generate cyclohexanone oxime. Therefore, the hydroxylamine oximation of cyclohexanone method has a long production process, large investment, complex operation and control, high hydrogen consumption and high material consumption, so the production cost is high.

To reduce the cost of oximation and improve an atom utilization of oximation, Ente Nazionale Idrocarburi developed the HAO method (US Patent Application Publication No. 474521A) and realized industrialization: one-step synthesis of cyclohexanone oxime by cyclohexanone, hydrogen peroxide solution and ammonia under the action of titanium silicalite molecular sieves. Compared to HSO method and HPO method, HAO method has advantages of low hydrogen consumption, short production process, simple control, low requirements for equipment and pipe materials and less investment and footprint. However, HAO method consumes hydrogen peroxide solution, so it’s necessary to be configured with a hydrogen peroxide solution production line. Besides, the hydrogen peroxide concentration should not be too high and the oximation process also produce water, so the method has large waste water and heavy treatment burden.

TORAY built and put into production a production unit of cyclohexanone oxime without cyclohexanone as intermediate in 1960s, which adopted photonitrosation of cyclohexane: a synthesis of cyclohexanone oxime hydrochloride by photochemical reaction of cyclohexane and nitrosyl chloride obtained by reaction of nitrosylsulfuric acid ($NOHSO_4$) and HCl. This method has advantages of less reaction steps and short process, but the power consumption is very high, the cost of light source equipment is high, the maintenance is relatively troublesome, and it has been shut down.

Actually, a synthesis of cyclohexanone oxime without cyclohexanone as intermediate was proposed in 1950s: a synthesis of partial oxidation of cyclohexanone oxime by oxidant such as hydrogen peroxide solution and peroxide (Journal of Molecular Catalysis A: Chemical, 2000, 160:393-402), which has been focused on since it was published. The earlier research focused on oxidants of hydrogen peroxide solution or alkyl hydrogen peroxide, as proposed in US Patent in US Patent Publication No. 2718528A (1955) and US Patent Publication No. 3960954A (1976). Later, the cost

of oxidants was taken into consideration and research emphasis were shifted to oxidants of molecular oxygen, for example, in 1982, Allied Corporation proposed a synthesis of cyclohexanone oxime by gas-solid phase catalytic oxidation of cyclohexylamine and molecular oxygen with a silica gel as catalyst at 150°C, and the selectivity of cyclohexanone oxime is 60% while the conversion efficiency of cyclohexylamine is 18% (US Patent Publication No.4337358A). In 1985, Allied Corporation proposed a synthesis of cyclohexanone oxime by gas-solid phase catalytic oxidation of cyclohexylamine and molecular oxygen with γ-aluminum oxide loaded with tungsten oxide as catalyst at 159°C, and the selectivity of cyclohexanone oxime is 54% while the conversion efficiency of cyclohexylamine is 28% (US Patent Publication No. 4504681A); the selectivity of cyclohexanone oxime can reach 64% while the conversion efficiency of cyclohexylamine is 33% if adopt γ-aluminium oxide loaded with molybdenum oxide as catalyst (J.of Catalysis, 1983, 83: 487-490). However, until the beginning of this century, the research results on synthesis of cyclohexanone oxime by partial oxidation of cyclohexylamine and molecular oxygen have made great process. For example, Chinese Patent Publication No. 103641740A(2013) published a gas phase catalytic oxidation with supported meso-porous silicon as catalyst, and the selectivity of cyclohexanone oxime can reach more than 85% while the conversion efficiency of cyclohexylamine is 20~30%; Chinese Patent Publication No. 109206339A(2017) published a liquid phase catalytic oxidation with supported titanium dioxide as catalyst, and the selectivity of cyclohexanone oxime can reach more than 90% while the conversion efficiency of cyclohexylamine is more than 50%.

Based on the above methods, AsahiKASEI proposed a new process for cyclohexanone oxime production (Chinese Patent No. 02804368.5 and Chinese Patent No. 02814607.7): firstly, cyclohexylamine is produced by amination of ammonia and cyclohexanol obtained by hydration of cyclohexene, then, cyclohexanone oxime is produced by partial oxidation of cyclohexylamine and molecular oxygen with catalyst. To obtain relatively high cyclohexanone oxime yield, the by-products (by-product-α and by-product-β) generated in the two-step need to be separated and recycled back into amination system to produce cyclohexylamine by amination with ammonia. This method has obvious advantages: first, the energy consumption is reduced because there is no need to dehydrogenate cyclohexanol to cyclohexanone; second, because there is no need for oximation of cyclohexanone, there is no need to consume hydroxylamine salt and hydrogen peroxide solution and their production lines. Therefore, the production cost of cyclohexanone oxime can be significantly reduced and it also has advantages of short process, less investment, less space occupation and easy operation control. However, there are also some disadvantages: first, the cyclohexanol is still produced by hydration of cyclohexene, so it still can not avoid the problem of high energy consumption as mentioned before; second, some by-products close to or higher than the boiling point of cyclohexanone oxime will be produced by amination of cyclohexanol and oxidation of cyclohexylamine, for example, the boiling point of nitrocyclohexane is 205~206°C, which is very close to the boiling point of cyclohexanone oxime 206~210°C, and boiling points of dicyclohexylamine and N-cyclohexyl hexamethyleneimine are higher than that of cyclohexanone oxime. Therefore, the separation of cyclohexanone oxime from such by-products with close or higher boiling points is extremely difficult and needs large energy consumption.

In addition, US Patent US 2967200A (1959) and US Patent US 3255261A (1964) proposed a method of a synthesis of cyclohexanone oxime without cyclohexanone as intermediate: nitrocyclohexane is produced by reaction of cyclohexane and nitric acid, and then cyclohexanone oxime is produced by partial hydrogenation of nitrocyclohexane. Although the procedure is simple, there are still many problems: the reaction condition of nitrification of cyclohexane and nitric acid is harsh (at temperature 150~200°C, pressure 3-4 MPa); equipment corrosion is serious and environment impact is relatively large; cyclohexane conversion is only about 20% and selectivity of nitrocyclohexane based on cyclohexane and nitric acid is less than 80%; and selectivity of cyclohexanone oxime by partial hydrogenation of nitrocyclohexane is less than 60%. However, until 21st century, the research on the synthesis and hydrogenation of nitrocyclohexane became active and made great progress. For example, Chinese Patent No. 101781217B proposed a highly selective co-production of nitrocyclohexane and adipic acid; Chinese Patent Publication No. 111530465A disclosed a supported porous carbon catalyst for hydrogenation of nitrocyclohexane, which conversion efficiency of nitrocyclohexane can reach 99%, and selectivities of cyclohexanone oxime and cyclohexylamine are about 90% and 10%, respectively.

In summary, with the constant development and progress of society, it is required to develop a simple, efficient and environmentally-friendly method for producing adipic acid and cyclohexanone oxime.

## SUMMARY

In view of the above problems of the prior art, especially high energy consumption, serious environmental pollution, and great difficulty in achieving the large-scale industrial production, this application provides a method for co-producing adipic acid and cyclohexanone oxime from cyclohexane with simple, efficient and environmentally-friendly operation.

The method for co-producing adipic acid and cyclohexanone oxime from cyclohexane includes two or three steps (depending on whether the cyclohexylamine obtained in step (2) is directly treated as a by-product or subsequently converted into cyclohexanone oxime):

(1) Oxidation-nitrification of cyclohexane
subjecting cyclohexane and $NO_x$ to catalytic or non-catalytic oxidation-nitrification reaction to produce a first reaction mixture containing adipic acid, nitrocyclohexane and a small amount of by-product A mainly including NO; and separating adipic acid and nitrocyclohexane from the first reaction mixture;
(2) Catalytic hydrogenation of nitrocyclohexane
subjecting the nitrocyclohexane obtained in the step (1) to catalytic hydrogenation with hydrogen to produce cyclohexanone oxime and cyclohexylamine; separating the cyclohexylamine from crude cyclohexanone oxime. Owing to a small amount and a certain economic value of cyclohexylamine, the cyclohexylamine can be treated as the by-product, or be partially oxidized to convert into cyclohexanone oxime in step (3);
(3) Partial oxidation of cyclohexylamine
subjecting the cyclohexylamine obtained in the step (2) molecular oxygen under the action of a catalyst to obtain a second reaction mixture containing cyclohexanone oxime and by-product B, and cyclohexylamine is absent or present in the second reaction mixture, then the second reaction mixture can be treated in one of the following ways:

(4-1) without separation, or after separating of part or all of water from the second reaction mixture, subjecting the second reaction mixture to simultaneous hydrogenation and amination in the presence of $H_2$ and $NH_3$ under the action of a catalyst, or sequentially to hydrogenation with $H_2$ and amination with $NH_3$ under the action of a catalyst, followed by separation to obtain cyclohexanone oxime; or
(4-2) without separation, or after removing part or all of water from the second reaction mixture by distillation, under the action of a catalyst, subjecting the second reaction mixture to hydrogenation with $H_2$, followed by separation to obtain cyclohexanone oxime.

In an embodiment, in the step (1), a total selectivity of adipic acid and nitrocyclohexane in the first reaction mixture is large than 80%; and a molar ratio of adipic acid to nitrocyclohexane in the first reaction mixture is 0.05-20: 1.
In an embodiment, the $NO_x$ can be one or more of $N_2O$, NO, $NO_2$, $N_2O_3$, $N_2O_4$ and $N_2O_5$, or a mixture of nitrogen oxides and molecular oxygen, and x represents a ratio of the number of O atoms to the number of N atoms in the $NO_x$.
In an embodiment, the by-product A is 1-nitro-1-cyclohexene, cyclohexyl nitrate, glutaric acid, succinic acid, cyclohexanone and cyclohexanol or a combination thereof; the by-product B can is water, hexamethyleneimine, cyclohexanone, nitrocyclohexane, N-cyclohexyl hexamethyleneimine and dicyclohexylamine or a combination thereof.
In an embodiment, in the step (2), a molar ratio of cyclohexanone oxime to cyclohexylamine is 2-50:1 in resultant products in the step (2).
In an embodiment, in the step (4-1), through the hydrogenation and amination simultaneously or sequentially performed under the action of the catalyst, the by-product B is converted into cyclohexylamine and cyclohexanone oxime; and in the step (4-2), through the hydrogenation with $H_2$ under the action of the catalyst, the by-product B is converted into a mixture of cyclohexylamine, cyclohexanone oxime, cyclohexanol and dicyclohexylamine.
In an embodiment, the simultaneous hydrogenation and amination, the sequential hydrogenation and amination, and the hydrogenation are coupled or not coupled with water separation.
In an embodiment, in the step (1), the oxidation-nitrification is performed in the presence of a solid catalyst, an inducer or a combination. An active component of the solid catalyst is vanadium-phosphorus-oxygen complex (VPO complex), imide compounds, zeolites or molecular sieve, solid acid, and Salen transition metal or heteropoly acid; and the inducer is selected from the group consisting of peroxide, alcohols, ketones compounds and esters.
In an embodiment, in the step (2), an active component of a catalyst for the catalytic hydrogenation is selected from one or more of Group VIII transition metals, and a promoter component is selected from one or more of Group IB~VIIB transition metals; the solid catalyst of partial oxidation of cyclohexylamine is surface hydroxyl-group-rich catalyst or supported catalyst.
In an embodiment, the catalyst used the simultaneous hydrogenation and amination or amination is a solid catalyst formed by compounding hydrotalcite or a hydrotalcite-like compound with a transition metal element active component, the transition metal element active component includes a main active component and a promoter component. The main active component is selected from one or more of the Group VIII transition metals, and the promoter component is selected from one or more of the Group IB~VIIB transition metals.
The present disclosure has the following beneficial effects.

(i) Adipic acid and cyclohexanone oxime are co-produced, and the ratio of adipic acid and cyclohexanone oxime can be adjusted within a certain range: subjecting cyclohexane and nitrogen oxides to simultaneous oxidation and nitrification to produce adipic acid and nitrocyclohexane; subjecting nitrocyclohexane to hydrogenation to directly produce cyclohexanone oxime and a small amount of cyclohexylamine. Owing to the small amount and economic value of cyclohexylamine, the cyclohexylamine can be treated as a by-product and be further converted into cyclohexanone oxime by partial oxidation.

(ii) A production of adipic acid has characteristics of shortened process, high atom utilization efficiency and low equipment investment: a one-step synthesis of adipic acid by oxidation of cyclohexane and $NO_x$. In this way, the yield of adipic acid converted by cyclohexane is high, and a large part of reactant $NO_x$ is converted into NO which is easy to recycle. Therefore, compared with existing traditional production of adipic acid by oxidation of KA oil with nitric acid in industry, the method omits production process of nitric acid and KA oil (or cyclohexanol), and saves a lot of energy consumption. Besides, because the utilization efficiency of N is greatly improved, the production of strong greenhouse gas $N_2O$ is significantly reduced.

(iii) A synthesis process of cyclohexanone oxime has characteristics of shortened process, low material consumption and low energy consumption: (1) A two-step or three-step synthesis of cyclohexanone oxime: subjecting cyclohexane and $NO_x$ to oxidation to produce adipic acid and nitration to produce nitrocyclohexane simultaneously, and nitrocyclohexane can produce cyclohexanone oxime and a small amount of cyclohexylamine by hydrogenation. Owing to the small amount and certainly economic value of cyclohexylamine, cyclohexylamine can be treated as the by-product and be converted into cyclohexanone oxime by partial oxidation. (2) Compared with existing industrial process of cyclohexanone oxime, the present disclosure avoids complicated processes of production of cyclohexanone so as to avoids their technological disadvantages of its oximation producing cyclohexanone oxime, such as: high energy consumption and waste lye of cyclohexanone by oxidation of cyclohexane, high energy of cyclohexanone by hydration of cyclohexene and consumption of hydroxylamine and hydrogen peroxide solution in cyclohexanone oxime production by oximation of cyclohexanone.

Therefore, compared with existing production technology of adipic acid and cyclohexanone oxime, a method for co-producing adipic acid and cyclohexanone oxime from cyclohexane of the present disclosure has shortened process, less construction investment, low energy consumption, simple and safe operation and environment-friendly process.

To understand easily, the above steps of the present disclosure are described below with reference to Fig. 1 (aiming at describing the present disclosure rather than limiting it).

(1) Oxidation-nitrification of cyclohexane: subjecting cyclohexane and $NO_x$ to catalytical or non-catalytical oxidation-nitrification to produce adipic acid and nitrocyclohexane. It also produces nitrogen oxides mainly contained NO and a small amount of by-product A. After separation, the obtained crude adipic acid is further separated and purified as a main-product; the obtained nitrocyclohexane goes to next step and is hydrogenated with a catalyst with hydrogen to produce another main-product cyclohexanone oxime; the obtained cyclohexane, $NO_x$ and nitrogen oxides such as NO generated by the reaction are recycled; the obtained by-product A can be treated as the by-product because of its small amount.

The $NO_x$ described herein represents nitrogen oxides (such as one of more of $N_2O$, NO, $NO_2$, $N_2O_3$, $N_2O_4$ and $N_2O_5$) and a mixture of nitrogen oxides and oxygen. Therefore, x represents a ratio of O to N, preferably x=1.5~4: 1, preferably x=2~3.5: 1.

The reactions of cyclohexane and $NO_x$ to produce adipic acid and nitrocyclohexane are as following stoichiometric equations:

$$C_6H_{12} + \frac{5}{x-1}NO_x \longrightarrow (CH_2)_4(COOH)_2 + H_2O + \frac{5}{x-1}NO \qquad \text{(f-1)}$$

$$2C_6H_{12} + \frac{3}{x-1}NO_x \longrightarrow 2C_6H_{11}NO_2 + H_2O + \frac{5-2x}{x-1}NO \qquad \text{(f-2)}$$

The reactions to produce by-products such as cyclohexyl nitrate, 1-nitro-1-cyclohexene, cyclohexanone, cyclohexanol and glutaric acid are as following stoichiometric equations:

$$2C_6H_{12} + \frac{5}{x-1}NO_x \longrightarrow 2C_6H_{11}ONO_2 + H_2O + \frac{7-2x}{x-1}NO \qquad \text{(f-3)}$$

$$2C_6H_{12} + \frac{5}{x-1}NO_x \longrightarrow 2C_6H_9NO_2 + 3H_2O + \frac{7-2x}{x-1}NO \qquad \text{(f-4)}$$

$$2C_6H_{12} + \frac{3}{x-1}NO_x \longrightarrow C_6H_{10}O + C_6H_{11}OH + H_2O + \frac{3}{x-1}NO \quad (f\text{-}5)$$

$$C_6H_{12} + \frac{8}{x-1}NO_x \longrightarrow (CH_2)_3(COOH)_2 + 2H_2O + CO_2 + \frac{8}{x-1}NO \quad (f\text{-}6)$$

It should be noted that above equations are stoichiometric equations from reactants, cyclohexane and $NO_x$, to target products or main by-products and NO rather than actual reaction processes or reaction equations. In fact, cyclohexanone and cyclohexanol produced in reaction may be further oxidized to adipic acid by $NO_x$, and nitrocyclohexene can also be further hydrogenated to obtain cyclohexanone oxime. Besides, the generated water may react with $NO_x$ to generate nitric acid, and $NO_x$ may participate in oxidation with cyclohexanol and cyclohexanone.

In the present disclosure, though main gas product produced in reactions is NO, a small amount of other nitrogen oxides, such as $N_2O$, are also be produced. Besides, less $CO_2$ is produced because the amount of glutaric acid and succinic acid produced in the present disclosure is very small.

As is known, NO produced in reactions is easily recycled after separation. NO is mixed with a certain proportion of $O_2$ according to requirements of reactants $NO_x$.

$$NO + \frac{x}{2}O_2 \longrightarrow NO_x \quad (f\text{-}7)$$

If a ratio of oxygen to nitrogen of raw reaction material $NO_x$ is moderate (the value of x is moderate), net production of NO generated in whole reaction process can be very low which is a content of NO in reaction products of oxidation and nitrification of cyclohexane can be very low.

The ratio of adipic acid to nitrocyclohexane, the target product of oxidation and nitrification of cyclohexane and $NO_x$ above, can be adjusted as required: by changing the composition and dosage of a solid catalyst, a ratio of cyclohexane to $NO_x$, reaction temperature and pressure and a molar ratio of adipic acid to nitrocyclohexane can be adjusted within the range of 1:10-10:1 or even 1:20-20:1. The solid catalyst includes VPO complexe, such as M-VPO and M-AlVPO (M is a transition metal, such as Mn, Cu, Co, Ni and Cr); imide compound, such as N-hydroxyphthalimide, 2,6-dihydroxypyrrolo(3,4-f)isoindole-1,3,5,7(2H,6H)-tetrone and N-hydroxy-1,8-naphthalimide; zeolite and molecular sieve, such as HZSM-5 molecular sieve, H-Y molecular sieve, β-zeolite and TS-1 molecular sieve; solid acid, such as sulfoacid resin, sulfuric acid/silica gel, $SO_4^{2-}/[TiO_2\ (4)\text{-}MoO_3\ (1)]$ and $SO_4^{2-}/ZrO_2\text{-}Ce_2O_3$; metal oxide, such as $TiO_2$, $V_2O_5$, $\gamma\text{-}Al_2O_3$, $ZrO_2$, NiO, CrO, $MnO_2$, CuO, $Ce_2O_3$,$WO_3$; Salen transition metal catalyst, such as cosalen, Cosalen/NaY, Cosalen/A1PO-5 and Cosalen/MCM-41; and heteropoly acid, such as HPAs and phosphotungstic acid. Inducer, such as peroxides, alcohol compound, ketones, aldehydes and esters can be added in this process. The dosage of the solid catalyst or the inducer is 0~0.3 times required mass of catalyst per unit target product (adipic acid and nitrocyclohexane), preferably 0.001-0.1:1; a molar ratio of cyclohexane to $NO_x$ is 0.1-20, preferably 0.2-6; reaction temperature is 10~300°C, preferably 40~200°C; and reaction pressure (absolute pressure) is 0.1~5MPa, preferably 0.2~1.5MPa.

(2) Hydrogenation of nitrocyclohexane: nitrocyclohexane obtained in last step is hydrogenated with hydrogen to main produce cyclohexanone oxime and cyclohexylamine. The stoichiometric equations are as follows:

$$C_6H_{11}NO_2 + H_2 \rightarrow (CH_2)_5NOH + H_2O \quad (f\text{-}8)$$

$$C_6H_{11}NO_2 + 3H_2 \rightarrow C_6H_{11}NH_2 + 2H_2O \quad (f\text{-}9)$$

In fact, if by-product A contains 1-nitro-1-cyclohexene, it can be separated form oxidation and nitrification with nitrocyclohexane, and 1-nitro-1-cyclohexene can be hydrogenated to cyclohexylamine and cyclohexanone oxime with nitrocyclohexane:

$$C_6H_9NO_2 + 2H_2 \rightarrow (CH_2)_5NOH + H_2O \quad (f\text{-}10)$$

$$C_6H_9NO_2 + 4H_2 \rightarrow C_6H_{11}NH_2 + 2H_2O \quad (f\text{-}11)$$

It shows that, hydrogenation of 1 mole of nitrocyclohexane requires only 1 mole of hydrogen, and hydrogenation of 1 mole of nitrocyclohexane requires 3 moles of hydrogen gas. Though nitrocyclohexane has a certain value, its market demand is much smaller than that of cyclohexanone oxime (a precursor of caprolactam). Therefore, in general, the molar

ratio of cyclohexanone oxime to cyclohexylamine in products should be increased as much as possible. However, if needed, in the present disclosure, a molar ration of cyclohexanone to cyclohexylamine, target products of hydrogenation, can be adjusted by changing composition and dosage of catalysts, a raw material ratio of nitrocyclohexane to hydrogen and reaction temperature and pressure.

In the present disclosure, an active component of solid catalyst for hydrogenation of nitrocyclohexane is selected from one or more of the Group VIII transition metals, such as cobalt, nickel, copper and palladium. The promoter component is selected from one or more of the Group IB~VIIB transition metals, such as copper and zinc; supporter includes carbon materials, such as activated carbon and carbon nanotubes. Reaction temperature is 40~300°C, preferably 60~200°C; and hydrogen pressure (absolute pressure) is 0.1-0.4MPa, preferably 0.2~2.0MPa.

Crude cyclohexanone oxime and cyclohexanamine are obtained by separating hydrogenated products. In general, because of a small amount of cyclohexanamine, it can be treated as the by-product, and it can also be partially oxidized with molecular oxygen and further converted into cyclohexanone oxime by amination and hydrogenation with $NH_3$ and $H_2$.

(3) Partial oxidation of cyclohexylamine: subjecting cyclohexylamine separated from last step to partial oxidation with molecular oxygen under the action of the solid catalyst to obtain a second reaction mixture containing cyclohexanone oxime and by-product B; without separation, or after separating part or all of water from the second reaction mixture, subjecting the second reaction mixture to simultaneous hydrogenation and amination in the presence of $H_2$ and $NH_3$ under the action of a catalyst, or sequentially to hydrogenation with $H_2$ and amination with $NH_3$ under the action of a catalyst, followed by separation to obtain cyclohexanone oxime; without separation, or after removing part or all of water from the second reaction mixture by distillation, under the action of a catalyst, subjecting the second reaction mixture to hydrogenation with $H_2$ followed by separation to obtain cyclohexanone oxime together with a small amount of dicyclohexylamine and cyclohexanol. Cyclohexanone oxime can be obtained by distilling and separating because boiling points of cyclohexylamine, cyclohexanol, dicyclohexylamine and cyclohexanone oxime differs relatively greatly (in particular to cyclohexylamine and cyclohexanone oxime differs greatly).

The by-product B mainly includes one or more of cyclohexanone, nitrocyclohexane, hexamethyleneimine and N-cyclohexyl hexamethyleneimine.

Main reactions of cyclohexylamine and molecular oxygen are as follows:

$$C_6H_{11}NH_2 + O_2 \rightarrow C_6H_{10}NOH + H_2O \quad \text{(f-12)}$$

$$C_6H_{11}NH_2 + 1.5O_2 \rightarrow C_6H_{11}NO_2 + H_2O \quad \text{(f-13)}$$

$$C_6H_{11}NH_2 + 0.5O_2 \rightarrow C_6H_{10}NH + H_2O \quad \text{(f-14)}$$

$$2C_6H_{11}NH_2 + 3.5O_2 \rightarrow 2C_6H_{10}O + 3H_2O + 2NO \quad \text{(f-15)}$$

$$C_6H_{10}O + C_6H_{11}NH_2 \rightarrow C_6H_{10}{=}N{-}C_6H_{11} + H_2O \quad \text{(f-16)}$$

$$2C_6H_{11}NH_2 + 2.5O_2 \rightarrow 2C_6H_{11}OH + H_2O + 2NO \quad \text{(f-17)}$$

$$C_6H_{11}OH + C_6H_{11}NH_2 \rightarrow (C_6H_{11})_2NH + H_2O \quad \text{(f-18).}$$

Referring to above reaction equations, by-reaction (f-15) and (f-17) that produce cyclohexanone and cyclohexanol should be strictly controlled for following reasons. They cause a loss of N and $NH_3$ by themselves, and produce N-cyclohexyl hexamethyleneimine or dicyclohexylamine to further cause the loss of N and $NH_3$ by consuming cyclohexylamine through reaction (f-16) and reaction (f-18). However, reaction rate of reaction (f-18) may faster than that of reaction (f-17), therefore, cyclohexanol is rarely found in the by-product B of the present disclosure.

Solid catalyst of partial oxidation of cyclohexylamine is selected from metal compounds, in which metal is form the Group IVB (Ti, Zr and Hf), or surface hydroxyl-group-rich catalyst, such as titanium dioxide, silica gel, aluminium oxide, VPO complex, metatitanic acid, metasilicic acid, tungsten trioxide or their supported catalysts. Taking $TiO_2$ or supported $TiO_2$/MCM-41 catalyst with hydroxyl-group-rich surface as an example, under reaction temperature of 100°C and oxygen pressure of 1.2MPa, the conversion efficiency of cyclohexylamine can reach above 40% and the selectivity of cyclohexanone oxime can reach 90%, and the rest products are cyclohexanon, nitrocyclohexane, hexamethyleneimine and N-cyclohexyl hexamethyleneimine.

In the present disclosure, the solid catalyst of partial oxidation of cyclohexylamine preferably has a hydroxyl-group-rich surface, which includes titanium dioxide, silica gel, aluminium oxide, VPO complex, metatitanic acid, metasilicic acid, tungsten trioxide or their supported catalysts. Reaction temperature is 50~200°C, preferably 70~150°C; oxygen pressure (absolute pressure) is 0.2~3.0MPa, preferably 0.4~2.0MPa.

However, a selectivity of the by-product B depends on a catalyst and reaction condition. A better selectivity may be about 5% and some catalysts can reach about 10%. If these by-products are not recycled, it will be a big problem in both economic and environmental terms. Therefore, AsahiKASEI (Chinese Patent No. 02814607.7) proposed that the by-product B is first separated from cyclohexanone oxime and then is returned to produce cyclohexylamine by amination with ammonia and hydrogen. However, in by-product B, except for cyclohexanone and hexamethyleneimine, boiling points of other substances are very close to or higher than the boiling point of cyclohexanone oxime. For example, the boiling point of nitrocyclohexane is 205-206°C which is close to the boiling point of 206-210°C of cyclohexanone oxime, and the boiling point of dicyclohexylamine and the boiling point of N-cyclohexyl hexamethyleneimine are above 255°C. It can be seen that it is difficult to separate them from cyclohexanone oxime, and the energy consumption of separation may be very high which will be greatly restricted in industrial application.

Relevant reactions of by-product B with hydrogen, ammonia or combination thereof are as follows:

$$C_6H_{10}O + H_2 \rightarrow C_6H_{11}OH \quad (f\text{-}19)$$

$$C_6H_{10}{=}N\text{-}C_6H_{11} + H_2 \rightarrow (C_6H_{11})_2NH \quad (f\text{-}20)$$

$$C_6H_{10}{=}NH + H_2 \rightleftarrows C_6H_{11}NH_2 \quad (f\text{-}21)$$

$$C_6H_{11}NO_2 + 3H_2 \rightarrow C_6H_{11}NH_2 + 2H_2O \quad (f\text{-}22)$$

$$C_6H_{11}NO_2 + H_2 \rightarrow C_6H_{10}NOH + H_2O \quad (f\text{-}23)$$

$$C_6H_{10}O + NH_3 + H_2 \rightarrow C_6H_{11}NH_2 + H_2O \quad (f\text{-}24)$$

$$C_6H_{10}{=}N\text{-}C_6H_{11} + NH_3 + H_2 \rightarrow 2C_6H_{11}NH_2 \quad (f\text{-}25)$$

$$(C_6H_{11})_2NH + NH_3 \rightarrow 2C_6H_{11}NH_2 \quad (f\text{-}26).$$

Catalysts used in the simultaneous hydrogenation and amination or amination in the present disclosure only catalyze the reaction conversion of by-product B with $H_2$, $NH_3$ or combination thereof and do not catalyze or hardly catalyze reaction conversion of cyclohexanone oxime with $H_2$, $NH_3$ or combination thereof; catalyst of the simultaneous hydrogenation and amination or amination is preferably formed by compounding hydrotalcite or a hydrotalcite-like compound with a transition metal element active component. The transition metal elemental active components include a main active component and a promoter component. The main active component is selected from one or more of the Group VIII transition metal, such as ferrum, nickel and platinum; the auxiliary active component is selected from one or more of the Group IB~VIIB transition metal, such as copper and zinc. The active component of hydrogenation catalysts is selected from one or more transition metal of the Group VIII transition metal, such as Ni, Co, Ru, Rh, Pt and Pd, and the auxiliary active component of hydrogenation catalysts is selected from one or more of the Group IB~VIIB transition metal, such as Cu, Zn, Zr and Mn.

Compared with existing methods for producing adipic acid and cyclohexanone oxime, the present disclosure mainly has following innovation points and benefits:

(i) The present disclosure can co-produce adipic acid and cyclohexanone oxime, and a mole ratio of adipic acid to cyclohexanone oxime can be adjusted in a relatively wide range. Therefore, flexible design of production equipment and appropriate adjustment in production process can be made according to market needs of adipic acid and cyclohexanone oxime, so that flexibility of industrial application and applied value of industrialization are improved.

(ii) In the present disclosure, a process of adipic acid production has shortened and environmentally-friendly process, and low material consumption and energy consumption. The present disclosure adopts $NO_x$ (x>1) to oxidize cyclohexane and synthesize adipic acid in one-step. Compared with the existing traditional production of adipic acid by oxidation of KA oil (from oxidation of cyclohexane or hydration of cyclohexene) with nitric acid in industry, the present disclosure simplifies production process greatly (omitting production process of nitric acid and KA oil) so that investment and space occupation are reduced greatly. It can also greatly improve adipic acid yield and greatly reduce energy and material consumption. In addition, reactant $NO_x$ is mainly converted into NO which is easily to form $NO_x$ with $O_2$, therefore, utilization of N has improved greatly and the production process is more friendly to

environment.

(iii) In the present disclosure, cyclohexanone oxime production has shortened process and low material and energy consumption. Most cyclohexanone oxime is synthesized by a two-step reaction from cyclohexane: cyclohexane is nitrated to nitrocyclohexane with $NO_x$, and then is hydrogenated to cyclohexanone oxime and a small amount of cyclohexylamine. Because of the small amount and a certain economic value of cyclohexanamine, it can be treated as the by-product, and it can also be partially oxidized with molecular oxygen and further converted into cyclohexanone oxime by amination and hydrogenation. Compared with existing industrial process of cyclohexanone oxime, the present disclosure avoids high material consumption of the KA oil production through cyclohexane oxidation and high material consumption of cyclohexanol production from cyclohexene hydration. With regard to the preparation of cyclohexanone oxime through oximation of cyclohexanone and hydroxylamine or ammoximation of cyclohexanone, the present disclosure does not require the consumption of hydroxylamine or hydrogen peroxide solution and corresponding production lines. In summary, the present disclosure has shortened and environmentally-friendly process, less equipment investment and space occupation, and low production cost.

## BRIEF DESCRIPTION OF THE DRAWINGS

Fig. 1 is a flow chart of a method for co-producing adipic acid and cyclohexanone oxime according to an embodiment of present disclosure.

## DETAILED DESCRIPTION OF EMBODIMENTS

The embodiments below are merely illustrative of, rather than limiting, the present disclosure.

(1) Oxidation and nitration of cyclohexane

### Example 1

A continuous gas phase reaction process in a fixed bed reactor was adopted. Liquid cyclohexane was input through a metering pump, and gasified in a preheating section. Then, the gasified cyclohexane was mixed with $NO_2$ in a molar ratio of 0.2:1, and introduced to a glass tube reactor with an inner diameter of φ10, in which a $V_2O_5$/MCM-41 catalyst with a height of about 10 cm was loaded. Temperature of the reactor was controlled at 180°C, and the outlet gas of the reactor was cooled by a glass condenser tube with a cooling jacket and discharged (the recirculating cooling water was kept at 5°C). The timing was started, and the condensate was collected after 2 hours of stable operation of the reaction system, and the input of cyclohexane and $NO_2$ into the reactor was stopped after 24-h continuous operation. After the reaction system was cooled to room temperature, products adhering to an outlet of the reactor and a wall of the condenser tube were quantitatively cleaned and collected with cyclohexane. The resultant product was filtered to collect a liquid phase (including unreacted cyclohexane, nitrocyclohexane and a small amount of by-products cyclohexyl nitrate and 1-nitro-1-cyclohexene, quantitatively analyzed by gas chromatography (GC) internal standard method) and a solid phase (including adipic acid and a small amount of by-products succinic acid and glutaric acid, quantitatively analyzed by high performance liquid chromatography (HPLC) external standard method). According to analysis results and material balance, the conversion rate of cyclohexane was 32.5%, and selectivities of adipic acid and nitrocyclohexane were 56.8% and 41.1%, respectively (with a total selectivity of 97.9%). Finally, the liquid phase was purified by distillation to obtain nitrocyclohexane with a purity of 98.6%, and the solid phase was purified by hot water dissolution and recrystallization to obtain adipic acid with a purity of 99.8%.

The process in Example 1 was repeated several times, and the resultant nitrocyclohexane was used in the subsequent hydrogenation example.

### Example 2

The reaction process was basically the same as that in Example 1, except that in this embodiment, $O_2$ was input in the reaction process, so that a ratio of cyclohexane:$NO_2$:$O_2$ was 0.8:1:0.1. According to analysis results and material balance of the liquid phase and solid phase products, the conversion rate of cyclohexane was 35.7%, and the selectivities of adipic acid and nitrocyclohexane were 50.2% and 48.4%, respectively (with a total selectivity of 98.6%). Finally, nitrocyclohexane with a purity of 98.4% was obtained from the separation and purification of the liquid phase, and adipic acid with a purity of 99.5% was obtained from separation and purification of the solid phase.

**Example 3**

A reaction process was the same as the example 1 with a difference that no catalyst was added. According to analysis results and material balance of all liquid phase and solid phase products, conversion rate of cyclohexane was 9.7% and the selectivities of adipic acid and nitrocyclohexane were 34.1% and 58.4%, respectively (total selectivity was 92.5%). Finally, nitrocyclohexane with a purity of 98.2% and adipic acid with a purity of 99.6% were obtained by separation and purification of liquid and solid phase.

**Example 4**

A batch reaction was adopted herein. To a 100 mL high-pressure reactor were added liquid cyclohexane and $NO_2$ in a molar ratio of 0.2:1 and 0.5 g of a Ni-VPO catalyst. After closing an inlet valve and an outlet valve, the reaction mixture was reacted at 90°C and 0.5 MPa under stirring for 2 h, and subjected to standing for layer separation. The upper liquid phase was quantitatively analyzed by GC internal standard method, and the bottom solid phase was quantitatively analyzed by LC external standard method. According to analysis results and material balance, the conversion rate of cyclohexane was 25.4%, and the selectivities of adipic acid and nitrocyclohexane were 71.2% and 24.1%, respectively (with a total selectivity of 95.3%). Finally, nitrocyclohexane with a purity of 98.6% and adipic acid with a purity of 99.9% were obtained by separation and purification as described in the Example 1.

**Example 5**

The process in this example was basically the same as Example 4, except that 0.1 MPa $O_2$ was introduced before the reaction. According to analysis results and material balance of the liquid phase and solid phase products, the conversion rate of cyclohexane was 28.2%, and the selectivities of adipic acid and nitrocyclohexane were 63.0% and 32.9%, respectively (with a total selectivity of 95.9%). Finally, nitrocyclohexane with a purity of 99.1% and adipic acid with a purity of 99.8% were obtained by separation and purification as described in the Example 1.

**Example 6**

A process was the same as the example 4 with a difference that no catalyst was added. According to analysis results and material balance of all liquid phase and solid phase products, the conversion rate of cyclohexane was 10.8% and the selectivities of adipic acid and nitrocyclohexane were 60.1% and 31.2%, respectively (total selectivity was 93.1%). Finally, nitrocyclohexane with a purity of 98.8% and adipic acid with a purity of 98.4% were obtained by separation and purification as described in the example 1.

(2) Hydrogenation of nitrocyclohexane

**Example 7**

To a 150 mL high-pressure reactor was added 0.3 g of 1%Cu-20%Ni/AC catalyst, and then air in the high-pressure reactor was replaced with hydrogen gas 4 times. The inlet and outlet valves were closed, and the high-pressure reactor was vacuumized with a vacuum pump. Then the inlet valve was opened, and 69.6 g of ethylenediamine (as solvent) and 12 g of nitrocyclohexane (with a purity of 98.6%) obtained in Example 1. The inlet valve was closed, and the high-pressure reactor was heated to 110°C, fed with hydrogen gas and maintained at 0.4 MPa. The reaction mixture was reacted under magnetic stirring for 6 h, cooled to room temperature and filtered to recycle the catalyst, and the resultant filtrate was analyzed by GC internal standard method. The conversion rate of nitrocyclohexane was 99.8%, a selectivity of cyclohexanone oxime was 89.7% and a selectivity of cyclohexylamine was 10.3%. The filtrate was subjected to distillation to obtain cyclohexylamine with a purity of 99.4% and cyclohexanone oxime with a purity of 99.6%.

This example was repeated several times, and the obtained cyclohexylamine was used in examples below.

(3) Partial oxidation of cyclohexylamine

**Example 8**

To a 100 mL reactor were added 18.5 g of the cyclohexylamine obtained in Example 7 and 0.4 g of a $WO_3/Al_2O_3$ catalyst. The reactor was fed with oxygen gas (with the pressure maintained at 1.0 MPa), and the reaction mixture was reacted at 110°C for 3 h, and filtered to recycle the solid catalyst. 20.6 g of the filtrate was obtained, and quantitatively analyzed by GC internal standard method. The conversion rate of cyclohexylamine was 40.6%; the selectivity of cyclohexanone oxime was 90.5%; a selectivity of nitrocyclohexane was 5.2%; a selectivity of cyclohexanone was 2.6%; a selectivity of hexamethyleneimine was 1.1%; and a selectivity of N-cyclohexyl hexamethyleneimine was 0.6%.

This example was repeated several times, and the resultant reaction solution was used in examples below, where the reaction solution included 53.0% by weight of cyclohexylamine, 37.4% by weight of cyclohexanone oxime, 2.5% by weight of nitrocyclohexane, 0.9% by weight of cyclohexanone, 0.4% by weight of hexamethyleneimine and 0.2% by weight of N-cyclohexyl hexamethyleneimine.

(4) Hydration and amination or amination of oxidation product

**Example 9**

To a 50 mL reactor were added 15.6 g of the oxidation reaction solution obtained according to the Example 8 and 0.12 g of a hydrotalcite-based Pd-Cu/MgAlO catalyst. In the presence of hydrogen, the reactor was fed with 0.11 MPa ammonia gas (with pressure maintained at 1.0 MPa), and the reaction mixture was reacted at 120°C for 4 h, and filtered to recycle the solid catalyst. 16.11 g of a filtrate was obtained and quantitatively analyzed by GC internal standard method. The analysis results demonstrated that there were 8.77 g of cyclohexylamine, 6.14 g of cyclohexanone oxime, 0.001 g of N-cyclohexyl hexamethyleneimine, and 0.03 g of dicyclohexylamine in the filtrate, and the cyclohexanone, nitrocyclohexane and hexamethyleneimine in the oxidation reaction solution were almost completely converted. Finally, 8.35 g of cyclohexylamine with a purity of 99.9% and 5.88 g of cyclohexanone oxime with a purity of 99.8% were obtained by distillation.

**Example 10**

To a 50 mL reactor were added 12.5g of the oxidation reaction solution obtained according to the example 8 and 0.12g of hydrotalcite-based Pt-Zn/MgAlO catalyst. Air in the reactor was replaced 3 times by inputting hydrogen gas to maintain hydrogen gas pressure at 1.0MPa. The reaction mixture was reacted at 120°C for 3h. After reacting, the solid catalyst was separated by filtration and 12.81g of mixed solution was obtained which was qualitatively analyzed by GC-MC and quantitatively analyzed by internal standard method in gas chromatography (with chlorobenzene as an internal standard substance) to obtain 6.7g of cyclohexylamine, 4.82g of cyclohexanone oxime, 0.12g of cyclohexanol and 0.03g of dicyclohexylamine. Finally, 6.5g of cyclohexylamine with a purity of 99.9% and 4.7g of cyclohexanone oxime with a purity of 99.8% were obtained by distillation.

**Example 11**

To a 50 mL reactor were added a liquid product and 0.12g of hydrotalcite-based Pt-Zn/MgAlO catalyst, where the liquid products were obtained by isolating 0.7g of water and 0.11g of cyclohexylamine from 12.8g of the liquid oxidation reaction products obtained according to the example 8. Air in the reactor was replaced 3 times by inputting hydrogen gas, then the hydrogen gas pressure was maintained at 1.0MPa. The reaction mixture was reacted at 120°C for 3h. After reaction, the solid catalyst was separated by filtration and 12.15g of mixed solution was obtained which was qualitatively analyzed by GC-MC and quantitatively analyzed by internal standard method in gas chromatography (with chlorobenzene as an internal standard substance) to obtain 6.73g of cyclohexylamine, 5.05g of cyclohexanone oxime, 0.12g of cyclohexanol and 0.03g of dicyclohexylamine. Finally, 6.5g of cyclohexylamine with a purity of 99.9% and 4.9g of cyclohexanone oxime with a purity of 99.8% were obtained by distillation.

## Claims

1. A method for producing adipic acid and cyclohexanone oxime from cyclohexane, comprising:

   (1) subjecting cyclohexane and $NO_x$ to catalytic or non-catalytic oxidation-nitration reaction to produce a first reaction mixture containing adipic acid, nitrocyclohexane and by-product A; and separating adipic acid and nitrocyclohexane from the first reaction mixture;
   (2) subjecting the nitrocyclohexane obtained in the step (1) to catalytic hydrogenation with hydrogen to produce cyclohexanone oxime and cyclohexylamine; and separating cyclohexanone oxime from cyclohexylamine;
   (3) subjecting the cyclohexylamine obtained in the step (2) to partial oxidation with molecular oxygen under the action of a catalyst to obtain a second reaction mixture containing cyclohexanone oxime and by-product B, wherein cyclohexylamine is absent or present in the second reaction mixture; and
   (4-1) without separation, or after separating part or all of water from the second reaction mixture, subjecting the

second reaction mixture to simultaneous hydrogenation and amination in the presence of $H_2$ and $NH_3$ under the action of a catalyst, or sequentially to hydrogenation with $H_2$ and amination with $NH_3$ under the action of a catalyst, followed by separation to obtain cyclohexanone oxime; or
(4-2) without separation, or after removing part or all of water from the second reaction mixture by distillation, under the action of a catalyst, subjecting the second reaction mixture to hydrogenation with $H_2$ followed by separation to obtain cyclohexanone oxime.

2. The method according to claim 1, **characterized in that** in the step (1), a total selectivity of adipic acid and nitrocyclohexane in the first reaction mixture is larger than 80%; and a molar ratio of adipic acid to nitrocyclohexane in the first reaction mixture is 0.05-20:1.

3. The method according to claim 1 or 2, **characterized in that** the $NO_x$ is one or more of $N_2O$, NO, $NO_2$ $N_2O_3$, $N_2O_4$ and $N_2O_5$, or a mixture of molecular oxygen and one or more of $N_2O$, NO, $NO_2$ $N_2O_3$, $N_2O_4$ and $N_2O_5$, wherein x represents a ratio of the number of O atoms to the number of N atoms in the $NO_x$.

4. The method according to claim 1, **characterized in that** the by-product A is 1-nitro-1-cyclohexene, cyclohexyl nitrate, glutaric acid, succinic acid, cyclohexanone, cyclohexanol or a combination thereof; and the by-product B is water, hexamethyleneimine, cyclohexanone, nitrocyclohexane, N-cyclohexyl hexamethyleneimine, dicyclohexylamine or a combination thereof.

5. The method according to claim 1, **characterized in that** in the step (2), a molar ratio of cyclohexanone oxime to cyclohexylamine in a hydrogenation product of nitrocyclohexane is 2-50:1.

6. The method according to claim 1, **characterized in that** in the step (4-1), the hydrogenation and amination are coupled or not coupled with water separation.

7. The method according to claim 1, **characterized in that** in the step (4-1), through the hydrogenation and amination simultaneously or sequentially performed under the action of the catalyst, the by-product B is converted into cyclohexylamine or cyclohexanone oxime; and
in step (4-2), through the hydrogenation with $H_2$ under the action of the catalyst, the by-product B is converted into a mixture of cyclohexylamine, dicyclohexylamine and cyclohexanol or a mixture of cyclohexanone oxime, dicyclohexylamine and cyclohexanol.

8. The method according to claim 1 or 2, **characterized in that** in the step (1), the oxidation-nitration reaction is performed in the presence of a solid catalyst, an inducer or a combination; an active component of the solid catalyst is vanadium-phosphorus-oxygen complex (VPO complex), imide compound, zeolite or molecular sieve, solid acid, Salen transition metal or heteropoly acid; and the inducer is selected from the group consisting of peroxides, alcohols, ketones and esters.

9. The method according to claim 1 or 7, **characterized in that** in the step (2), an active component of a catalyst for the catalytic hydrogenation is selected from one or more of Group-VIII transition metals, and a promoter component of the catalyst for the catalytic hydrogenation is selected from one or more of Group IB~VIIB transition metals; and in step (3), the catalyst used in the partial oxidation is a surface hydroxyl-rich catalyst or its supported catalyst.

10. The method according to claim 1 or 7, **characterized in that** in the step (4-1), the catalyst used in the simultaneous hydrogenation and amination or amination is a solid catalyst formed by compounding hydrotalcite or a hydrotalcite-like compound with a transition metal element active component; the transition metal element active component comprises a main active component and an auxiliary active component; the main active component is selected from one or more of Group-VIII transition metals; and the auxiliary active component is selected from one or more of Group IB~VIIB transition metals.

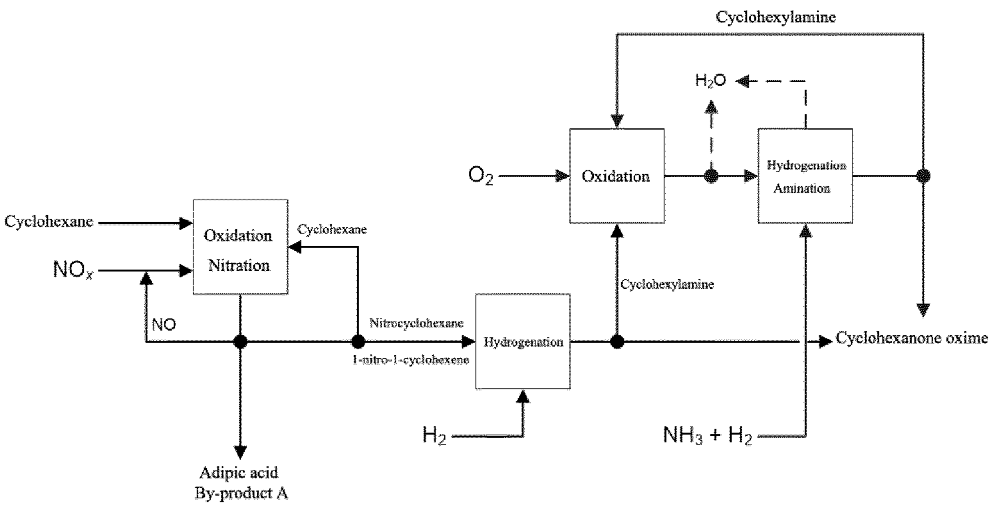
Cyclohexylamine
$H_2O$
$O_2$
Oxidation
Hydrogenation
Amination
Cyclohexane
$NO_x$
Oxidation
Nitration
Cyclohexane
NO
Nitrocyclohexane
1-nitro-1-cyclohexene
Hydrogenation
Cyclohexylamine
Cyclohexanone oxime
$H_2$
$NH_3 + H_2$
Adipic acid
By-product A

**Fig. 1**

**INTERNATIONAL SEARCH REPORT**

International application No.
**PCT/CN2021/130661**

**A. CLASSIFICATION OF SUBJECT MATTER**

C07C 51/27(2006.01)i; C07C 249/10(2006.01)i; C07C 55/14(2006.01)n; C07C 251/44(2006.01)n

According to International Patent Classification (IPC) or to both national classification and IPC

**B. FIELDS SEARCHED**

Minimum documentation searched (classification system followed by classification symbols)

C07C 51/-; C07C 55/-; C07C 249/-; C07C 251/-

Documentation searched other than minimum documentation to the extent that such documents are included in the fields searched

Electronic data base consulted during the international search (name of data base and, where practicable, search terms used)

CNTXT, WPABSC, CNKI, REGISTRY, CAPLUS: 湘潭大学, 罗和安, 游奎一, 蹇建, 刘平乐, 倪文金, 艾秋红, 已二酸, 环已酮肟, 环已烷, 硝基环已烷, 环已胺, 110-82-7, 124-04-9, 1122-60-7, 100-64-1, 108-91-8, adipic acid, cyclohexane, cyclohexanone oxime

**C. DOCUMENTS CONSIDERED TO BE RELEVANT**

| Category* | Citation of document, with indication, where appropriate, of the relevant passages | Relevant to claim No. |
|---|---|---|
| PX | CN 112939765 A (XIANGTAN UNIVERSITY) 11 June 2021 (2021-06-11)<br>claims 1-10 | 1-10 |
| Y | CN 103288626 A (XIANGTAN UNIVERSITY) 11 September 2013 (2013-09-11)<br>description paragraphs 11, 20, 27, 30-31, 61 | 1-10 |
| Y | 毛丽秋 等 (MAO, Liqiu et al.). "Pd/C催化硝基环已烷氢化制备环已酮肟 (Hydrogenation of Nitrocyclohexane to Cyclohexanone Oxime Catalyzed by Pd/C)"<br>*化工进展 (Chemical Industry and Engineering Progress),*<br>Vol. 28, No. 6, 30 June 2009 (2009-06-30),<br>ISSN: 1000-6613,<br>abstract, sections 1.2 and 1.3 | 1-10 |
| Y | CN 111253281 A (XIANGTAN UNIVERSITY) 09 June 2020 (2020-06-09)<br>abstract, description paragraphs 20, 23, 25-26 | 1-10 |
| Y | CN 101781217 A (XIANGTAN UNIVERSITY) 21 July 2010 (2010-07-21)<br>abstract, and claims | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2022** | **16 February 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/130661**

| C. DOCUMENTS CONSIDERED TO BE RELEVANT | | |
|---|---|---|
| Category* | Citation of document, with indication, where appropriate, of the relevant passages | Relevant to claim No. |
| A | GB 908757 A (E. I. DU PONT DE NEMOURS AND COMPANY) 24 October 1962 (1962-10-24)<br>entire document | 1-10 |
| A | 蹇建 等 (JIAN, Jian et al.). "NO2选择性催化氧化环己烷一步高选择性制备己二酸 (Non-official translation: Single-stage Preparation of Adipic Acid at High Selectivity from Selective Catalytic Oxidation of Cyclohexene with NO2)"<br>*第九届全国环境催化与环境材料学术会议——助力两型社会快速发展的环境催化与环境材料会议论文集 (Non-official translation: Proceedings of the 9th National Conference on Environmental Catalysis and Materials of China: Conference on Environmental Catalysis and Materials for Supporting Rapid Development of Two-type Society ),*<br>30 November 2015 (2015-11-30),<br>entire document | 1-10 |
| A | HILLS P.R. et al. "Radiation-initiated nitrosation of cyclohexane"<br>*International Journal of Applied Radiation and Isotopes,*<br>Vol. 12, No. 3-4, 09 October 2002 (2002-10-09),<br>ISSN: 0020-708X,<br>pp. 80-86 | 1-10 |
| A | MATSUMOTO Y. et al. "Metal-Free Synthesis of Adipic Acid via Organocatalytic Direct Oxidation of Cyclohexane under Ambient Temperature and Pressure"<br>*Organic Process Research & Development,* Vol. 22, No. 9, 07 September 2018 (2018-09-07),<br>ISSN: 1083-6160,<br>pp. 1312-1317 | 1-10 |

Form PCT/ISA/210 (second sheet) (January 2015)

**INTERNATIONAL SEARCH REPORT**
**Information on patent family members**

International application No.
**PCT/CN2021/130661**

| Patent document cited in search report | Publication date (day/month/year) | Patent family member(s) | Publication date (day/month/year) |
|---|---|---|---|
| CN 112939765 A | 11 June 2021 | None | |
| CN 103288626 A | 11 September 2013 | WO 2014202031 A8 | 31 March 2016 |
| | | EP 3012243 A1 | 27 April 2016 |
| | | CN 103288626 B | 10 December 2014 |
| | | WO 2014202031 A1 | 24 December 2014 |
| CN 111253281 A | 09 June 2020 | None | |
| CN 101781217 A | 21 July 2010 | CN 101781217 B | 29 May 2013 |
| GB 908757 A | 24 October 1962 | US 3170952 A | 23 February 1965 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2223493 A
- US 4263453 A
- US 5321157 A
- FR 2722783 B
- FR 2746671 B
- US 0147777 A1
- US 7507856 B2
- US 0095258 A1
- CN 02804368
- CN 02814607
- US 474521 A
- US 2718528 A
- US 3960954 A
- US 4337358 A
- US 4504681 A
- CN 103641740 A
- CN 109206339 A
- US 2967200 A
- US 3255261 A
- CN 101781217 B
- CN 111530465 A


### Non-patent literature cited in the description


- **RAJA ; SANKAR ; THOMAS.** *J. Am. Chem. Soc.,* 1999
- **YUAN et al.** *Organic Process R&D,* 2004
- **LV ; REN ; LIU.** *Applied Catalysis A,* 2012
- *Journal of Molecular Catalysis A: Chemical,* 2000, vol. 160, 393-402
- *J.of Catalysis,* 1983, vol. 83, 487-490